# EUROPEAN PATENT SPECIFICATION

(11) **EP 4 460 260 B1**
(45) Date of publication and mention of the grant of the patent: **15.04.2026**
(21) Application number: 22854291.6
(22) Date of filing: 22.12.2022
(51) Int. Cl.: A61B 90/50, A61B 34/30, A61B 17/42

(54) **ADJUSTMENT DEVICES FOR POSITIONING A UTERINE MANIPULATOR**
EINSTELLVORRICHTUNGEN ZUR POSITIONIERUNG EINES GEBÄRMUTTERMANIPULATORS
DISPOSITIFS DE RÉGLAGE POUR POSITIONNER UN MANIPULATEUR UTÉRIN

(30) Priority: 03.01.2022 US 202263295981 P
(43) Date of publication of application: 13.11.2024
(73) Proprietor: CooperSurgical, Inc., Trumbull, CT 06611 (US)
(72) Inventor: IVOSEVIC, Milan, Kinnelon, New Jersey 07405 (US); PARADISE, Charles, Brooklyn, New York 11217 (US); SOTO, Orlando, Amesbury, Massachusetts 01913 (US); RODRIGUEZ, Christopher, Carlsbad, California 92009 (US); FALKENBURG, Cameron, San Diego, California 92117 (US); CROSS, Laura, Columbia, Maryland 21045 (US); MURPHY, Brian, Baltimore, Maryland 21218 (US); CHAUHAN, Raghuraj, Elkridge, Maryland 21075 (US)
(74) Representative: Fish & Richardson P.C.
(86) International application number: PCT/US2022/053836
(87) International publication number: WO 2023/129478

(56) References cited:
- EP-A1- 3 636 190
- EP-A1- 4 295 793
- EP-A1- 4 559 417
- WO-A1-2018/013965
- WO-A1-2020/206297
- WO-A1-2021/071540
- US-A- 5 554 160
- US-A1- 2020 253 676
- US-A1- 2021 307 850
- US-A1- 2021 369 367

## Description

### CROSS REFERENCE TO RELATED APPLICATION

This application claims priority to U.S. Provisional Patent Application No. 63/295,981, filed on January 03, 2022, pursuant to 35 USC §119.

### TECHNICAL FIELD

This disclosure relates to adjustment devices for positioning a uterine manipulator during a surgical procedure.

### BACKGROUND

Uterine manipulators are medical instruments that are used for manipulating a patient's uterus during surgical procedures, such as a laparoscopic gynecologic surgery (e.g., a total laparoscopic hysterectomy (TLH) surgery). With a distal portion of a uterine manipulator advanced through the vaginal canal and into the uterus, the uterus can be manipulated through surgeon-controlled movements of a proximal portion of the uterine manipulator. The uterine manipulator may be securely positioned and held in place during the procedure by a uterine positioning system that is mounted to an operating table. During such a procedure, a surgeon often needs to slightly adjust the position of the uterus via the uterine manipulator, requiring the surgeon either to leave his or her workstation to reposition the manipulator or to ask an assistant to do the same. In either case, the procedure is interrupted. Furthermore, if the surgeon asks an assistant to perform the adjustment, the situation is further complicated because the adjustment is blind and based only on verbal instructions from the surgeon.

US 2021/307850 A1 describes systems and methods for robotic manipulation of a patient's anatomy, such as the uterus, during minimally invasive surgical procedures.

WO 2018/013965 A1 describes a teleoperational medical system that comprises an input device and a manipulator configured to couple with and move an instrument. The system also comprises a control system including one or more processors. In response to a determination that the instrument is inserted into an instrument workspace in a corresponding direction to a field of view of the workspace, the control system is configured to map movement of the input device to movement of the instrument according to a first mapping. In response to a determination that the instrument is inserted into the instrument workspace in a non-corresponding direction to the field of view, the control system is configured to map movement of the input device to movement of the instrument according to a second mapping. The second mapping includes an inversion of the first mapping for at least one direction of motion of the instrument.

US 5 554 160 A describes a uterus maneuvering method involving the use of a lengthwise elongated boom, and a strut carried at one end of the boom to be pivoted relative to the boom, the strut and boom adapted to be inserted via the vaginal canal to locate the strut to project in the uterine cavity; actuator structure associated with the boom and strut, manipulable for effecting controlled pivoting of the strut; and control structure manipulable for controlling the actuator structure, and including a servo system having an extra-corporeal manual control structure, for coupling between the manual control structure and actuator structure, the coupling including electrical signal transmission structure.

### SUMMARY

An adjustment device and a positioning system according to the present invention are defined in claims 1 and 10, respectively. Further advantageous developments of the present invention are set out in the dependent claims.

In general, this disclosure relates to adjustment devices for positioning a uterine manipulator during a surgical procedure.

In one aspect of the present disclosure which is not claimed, an adjustment device for positioning a uterine manipulator includes a support component that is movable along an arcuate path defined by the adjustment device and an adjustment unit. The adjustment unit includes a base component that is secured to the support component and a rotational element that is secured to the base component and to the uterine manipulator such that the rotational element and the uterine manipulator secured thereto are movable along the arcuate path and rotatable with respect to the support component.

Examples may include one or more of the following features.

In some examples, the adjustment device is configured to be fixedly attached to a positioning arm of a uterine positioning system.

In some examples, the adjustment unit is configured to move forward and backward along the arcuate path with respect to the positioning arm.

In some examples, the adjustment device further includes internal electronics that are configured to effect wireless or wired communication with a remote controller.

In some examples, the adjustment device further includes a remote control unit that is configured to power and control one or more movement mechanisms of the adjustment device.

In some examples, the adjustment device further includes a support unit that provides an elongate receptacle, the elongate receptacle receiving the support component and defining the arcuate path.

In some examples, the support component includes a connector having an end that is retained within the elongate receptacle as the adjustment unit moves arcuately along the elongate receptacle.

In some examples, the adjustment unit is movable forward and backward along the arcuate path by a total distance of up to about 6 cm to up to about 26 cm.

In some examples, the rotational element includes a dial that is rotatable about a pin, and an angular span of the dial corresponds to a lateral range of a tip of the uterine manipulator of about 6 cm to about 36 cm.

In some examples, the support component defines the arcuate path.

In some examples, the adjustment device further includes one or more motors that control arcuate movement of the support component and rotation of the rotational element.

In some examples, the adjustment device further includes a translation assembly that is coupled to and causes linear movement of the support component.

In some examples, the adjustment device is configured to maintain rotation of a shaft of the uterine manipulator around a reference point as the support component moves along the arcuate path.

In some examples, the reference point is located at a patient's cervix.

In some examples, the adjustment device is configured such that rotation of the uterine manipulator by the rotational element is maintained about a reference point.

In some examples, the reference point is located at a patient's cervix, and wherein an axis of the adjustment unit intersects the reference point.

In another aspect of the present disclosure which is not claimed, a positioning system includes a positioning arm and an adjustment device supported on the positioning arm for positioning a uterine manipulator. The adjustment device includes a support component that is movable along an arcuate path defined by the adjustment device and an adjustment unit. The adjustment unit includes a base component that is secured to the support component and a rotational element that is secured to the base component and to the uterine manipulator such that the rotational element and the uterine manipulator secured thereto are movable along the arcuate path and rotatable with respect to the support component.

Examples may include one or more of the following features.

In some examples, the positioning arm defines a global position of the adjustment device and wherein the adjustment device defines a local position of the uterine manipulator.

In some examples, the positioning system further includes a remote controller and a control box that communicates with the remote controller.

In another aspect according to the present invention, an adjustment device for positioning a uterine manipulator includes a rotatable support component that is secured to the uterine manipulator and rotatable about a rotational axis defined by the adjustment device. The adjustment device further includes a rotatable adjustment unit that is secured to an articulation control element of the uterine manipulator and to the rotatable support component such that the adjustment device is configured to rotate the uterine manipulator with respect to the rotational axis and to articulate a portion of the uterine manipulator with respect to the rotatable support component.

The rotatable support component is coupled to a linear translation assembly.

The linear translation assembly includes a rail and a carriage.

The rotational axis intersects a patient's cervix.

Embodiments may include one or more of the following features.

In some embodiments, the linear translation assembly is configured to be fixedly attached to a positioning arm of a uterine positioning system.

In some embodiments, the adjustment device further includes one or more mechanical actuation cables that are configured to selectively cause the rotatable support component to rotate in opposite rotational directions.

In some embodiments, the adjustment device further includes a remote motor that is coupled to and controls the one or more mechanical actuation cables.

In some embodiments, the adjustment device further includes one or more mechanical actuation cables that are configured to selectively cause the rotatable adjustment unit to rotate in opposite rotational directions.

In some embodiments, the adjustment device further includes a remote motor that is coupled to and controls the one or more mechanical actuation cables.

In some embodiments, the articulation control element includes a handle and wherein the portion includes a distal tip.

In another aspect according to the present invention, a positioning system includes a positioning arm and an adjustment device supported on the positioning arm for positioning a uterine manipulator. The adjustment device includes a rotatable support component that is secured to the uterine manipulator and rotatable about a rotational axis defined by the adjustment device. The adjustment device further includes a rotatable adjustment unit that is secured to an articulation control element of the uterine manipulator and to the rotatable support component such that the adjustment device is configured to rotate the uterine manipulator with respect to the rotational axis and to articulate a portion of the uterine manipulator with respect to the rotatable support component.

In one aspect of the present disclosure which is not claimed, an adjustment device for positioning a uterine manipulator includes a base, an adjustment unit, and an adapter. The base includes an elongate receptacle, and the adjustment unit is movable arcuately along the elongate receptacle. The adapter is secured to a rotational element of the adjustment unit and to the uterine manipulator such that arcuate movement of the adjustment unit and rotational movement of the rotational element respectively effect arcuate movement and rotational movement of the uterine manipulator.

Examples may include one or more of the following features.

In some examples, the base is fixedly attachable to a positioning arm of a positioning system.

In some examples, the elongate receptacle has a curved profile.

In some examples, the arcuate movement of the adjustment unit includes a horizontal component.

In some examples, the adjustment unit is configured to move forward and backward along the elongate receptacle.

In some examples, the adjustment unit is movable forward and backward by a total distance of up to about 6 cm to up to about 26 cm.

In some examples, the adjustment unit includes a connector having an end that is retained within the elongate receptacle of the base as the adjustment unit moves arcuately along the elongate receptacle.

In some examples, the rotational element includes a dial that is rotatable about an interior pin.

In some examples, the dial is rotatable by an angle of up to about 90 degrees to up to about 270 degrees.

In some examples, an angular span of the dial corresponds to a lateral range of a tip of the uterine manipulator of about 6 cm to about 36 cm.

In some examples, the adjustment device further includes internal electronics housed within one or both of the base and the adjustment unit.

In some examples, the internal electronics facilitate wireless communication with a remote controller.

In some examples, the internal electronics facilitate wired communication with a remote controller.

In another aspect of the present disclosure which is not claimed, a positioning system includes a positioning arm and an adjustment device supported on the positioning arm for positioning a uterine manipulator. The adjustment device includes a base, an adjustment unit, and an adapter. The base includes an elongate receptacle, and the adjustment unit is movable arcuately along the elongate receptacle. The adapter is secured to a rotational element of the adjustment unit and to the uterine manipulator such that arcuate movement of the adjustment unit and rotational movement of the rotational element respectively effect arcuate movement and rotational movement of the uterine manipulator.

Examples may include one or more of the following features.

In some examples, the positioning arm defines a global position of the adjustment device.

In some examples, the positioning system further includes a remote controller.

In some examples, the adjustment device further included internal electronics that effect wireless communication with the remote controller.

In some examples, the internal electronics and a signal cable together effect wired communication with the remote controller.

In some examples, the adjustment unit is configured to move forward and backward along the elongate receptacle.

In some examples, the rotational element includes a dial that is rotatable about an interior pin.

Examples may provide one or more of the following advantages.

In some examples, remotely controlling robotic-like movements of the adjustment device can allow a surgeon to position a patient's uterus as desired for optimal cutting and removal access to the uterus without leaving his or her robotic surgery workstation at which a remote controller is operated. Thus, the surgeon can continue visualizing the patient's pelvic region on a display screen at the workstation while the adjustment device is remotely manipulated and without disruption. Such remote control of the adjustment device therefore advantageously results in a streamlined procedure with an improved effectiveness, an improved precision, and a reduced procedural time period.

Other aspects, features, and advantages will be apparent from the description, the drawings, and the claims.

### DESCRIPTION OF DRAWINGS

FIG. 1 is a perspective view of a positioning system, according to a comparative example, that supports a uterine manipulator.
FIG. 2 is a perspective view of an adjustment device of the positioning system of FIG. 1.
FIG. 3 is a side view of the adjustment device of FIG. 2.
FIG. 4 is a side view of the adjustment device of FIG. 2 with an adjustment unit illustrated in three different arcuate positions.
FIG. 5 is a perspective view of the adjustment device of FIG. 2 illustrated with a rotational element in three different angular positions.
FIG. 6 is a perspective view of an alternative uterine manipulator that can be supported by the positioning system of FIG. 1.
FIG. 7 is a side view of an adjustment device, according to a comparative example, in a reference linear configuration.
FIG. 8 is a side view of the adjustment device of FIG. 7 in an extended linear configuration.
FIG. 9 is a side view of the adjustment device of FIG. 7 in a reference arcuate configuration.
FIG. 10 is a side view of the adjustment device of FIG. 7 in an adjusted arcuate configuration.
FIG. 11 is a perspective view of the adjustment device of FIG. 7 in a first angular configuration.
FIG. 12 is a side view of the adjustment device of FIG. 7 in a second, opposite angular configuration.
FIG. 13 is a side view of an adjustment device, according to the present invention, in a reference linear configuration.
FIG. 14 is a side view of the adjustment device of FIG. 13 in an extended linear configuration.
FIG. 15 is a side view of the adjustment device of FIG. 13 in a first rotational configuration.
FIG. 16 is a side view of the adjustment device of FIG. 13 in a second rotational configuration that is opposite to the first rotational configuration.
FIG. 17 is a side view of the adjustment device of FIG. 13 in a third rotational configuration.
FIG. 18 is a side view of the adjustment device of FIG. 13 in a fourth rotational configuration that is opposite to the fourth rotational configuration.

### DETAILED DESCRIPTION

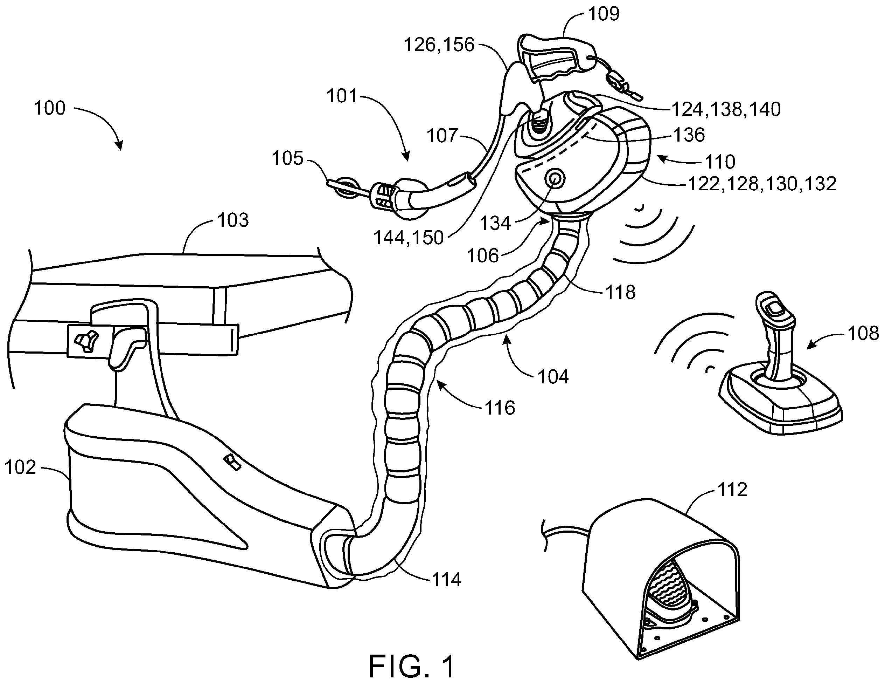

FIG. 1 illustrates a positioning system 100 that is designed to position a surgical tool for carrying out a surgical procedure. In some examples, the surgical procedure is a laparoscopic or robotic pelvic surgery for carrying out a total laparoscopic hysterectomy (TLH), and the surgical tool is a uterine manipulator 101 used to perform the TLH. The positioning system 100 is designed to be mounted to an operating table 103 and accordingly includes a mounting/powering unit 102. The positioning system 100 further includes a positioning arm 104 that extends from the mounting/powering unit 102, an adjustment device 110 that is attached to an upper end 106 of the positioning arm 104, a remote controller 108 for controlling certain components of the adjustment device 110, and a foot pedal 112 for locking and unlocking a configuration of the positioning arm 104.

The positioning arm 104 stably supports and securely positions the uterine manipulator 101 in place during the surgical procedure. The positioning arm 104 is embodied as a "gooseneck" structure that includes a rigid elbow section 114 and an adjustable section 116 to which the adjustment device 110 is attached. The adjustable section 116 includes multiple collars 118 (e.g., metal links) that are movable with respect to each other to provide the adjustable section 116 with a full range of motion. The positioning arm 104 also includes an internal tension cable (e.g., a braided mechanical cable) that extends from the upper end 106 of the positioning arm to the mounting/powering unit 102.

The foot pedal 112 can be depressed to unlock the positioning arm 104. That is, depression of the foot pedal 112 releases tension from the internal tension cable (e.g., deactivates the internal tension cable) to allow manual manipulation of the adjustable section 116 when desired. Conversely, the foot pedal 112 can be released from a depressed position to tension the internal tension cable (e.g., to activate the internal tension cable) for locking the configuration of the adjustable section 116. In the locked configuration, the collars 118 are positionally fixed, and the positioning arm 104 is rigid and immovable during the surgical procedure. Thus, in the locked configuration, the positioning arm 104 establishes a global position of the adjustment device 110, which is secured to its upper end 106. For locking and unlocking the positioning arm 104, the mounting/powering unit 102 includes a servo motor that is operable to pull and release the internal tension cable within the positioning arm 104. The mounting/powering unit 102 is powered via a wired connection to a wall plug.

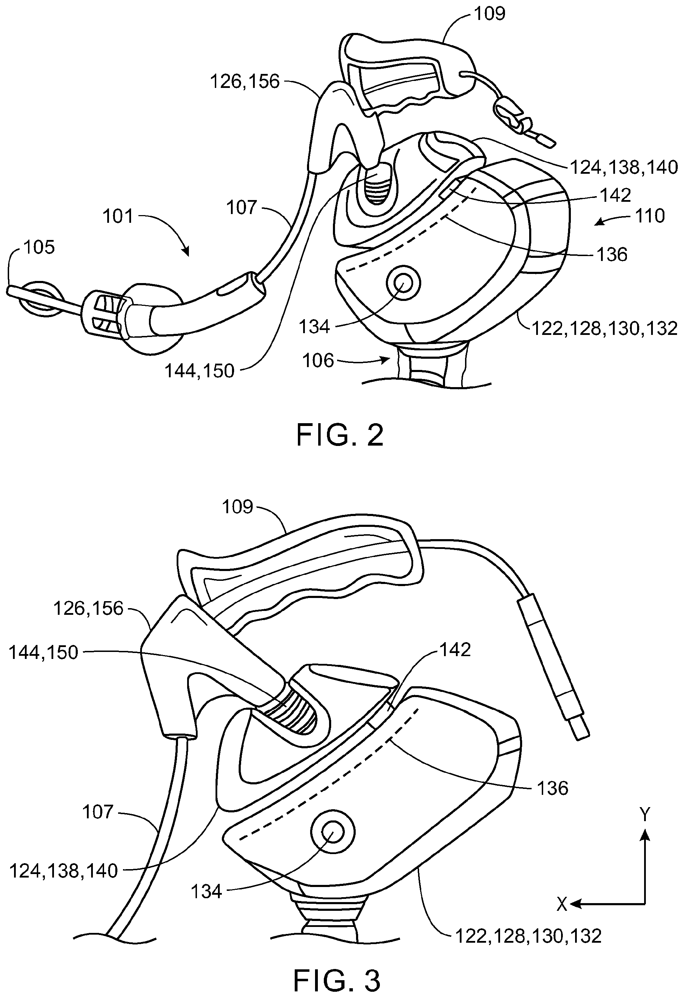

Referring to FIGS. 2 and 3, the adjustment device 110 is a robotic head that can further position (e.g., locally position) the uterine manipulator 101 with respect to the global position of the adjustment device 110. For example, once the positioning arm 104 has been locked in a desired configuration to establish a global position of the adjustment device 110, the adjustment device 110 can be operated with the remote controller 108 to fine-tune a position of the uterine manipulator 101, which is supported on the adjustment device 110. The adjustment device 110 includes a lower base 122 (e.g., a support unit) that is fixedly attached to the upper end 106 of the positioning arm 104, an upper adjustment unit 124 that is movable with respect to the base 122, and an adapter 126 that securely couples the adjustment unit 124 to the uterine manipulator 101.

The base 122 includes a housing 128, internal electronics 130, a rechargeable battery 132 that may be enclosed within the housing 128 for powering the adjustment device 110, and a power indicator light. Additionally, the base 122 may be connected directly to the remote controller 108 with a signal cable. The internal electronics 130 can effect wireless communication with the remote controller 108 and/or with the adjustment unit 124, as well as wired communication with the remote controller 108. The housing 128 carries a power selector 134 (e.g., a button or a switch) for powering the adjustment device 110 on and off. The base 122 further includes an elongate receptacle 136 (e.g., a rail or a slot indicated schematically with a dashed line that corresponds to an arcuate path) with a curved (e.g., arcuate) profile such that the adjustment unit 124 can undergo arcuate motion (e.g., curvilinear motion) in an *xy* plane along the elongate receptacle 136.

The adjustment unit 124 includes a housing 138 (e.g., a base component), internal electronics 140, a connector 142 (e.g., a support component) that moves along the elongate receptacle of the base 122, and a rotational element 144 (e.g., a pivotable element) that effectively allows the adapter 126 and the attached uterine manipulator 101 to be tilted from side to side. The internal electronics 140 can effect wireless communication with the remote controller 108 and/or with the base 122. In some examples, the internal electronics 140 can also effect wired communication with the remote controller 108.

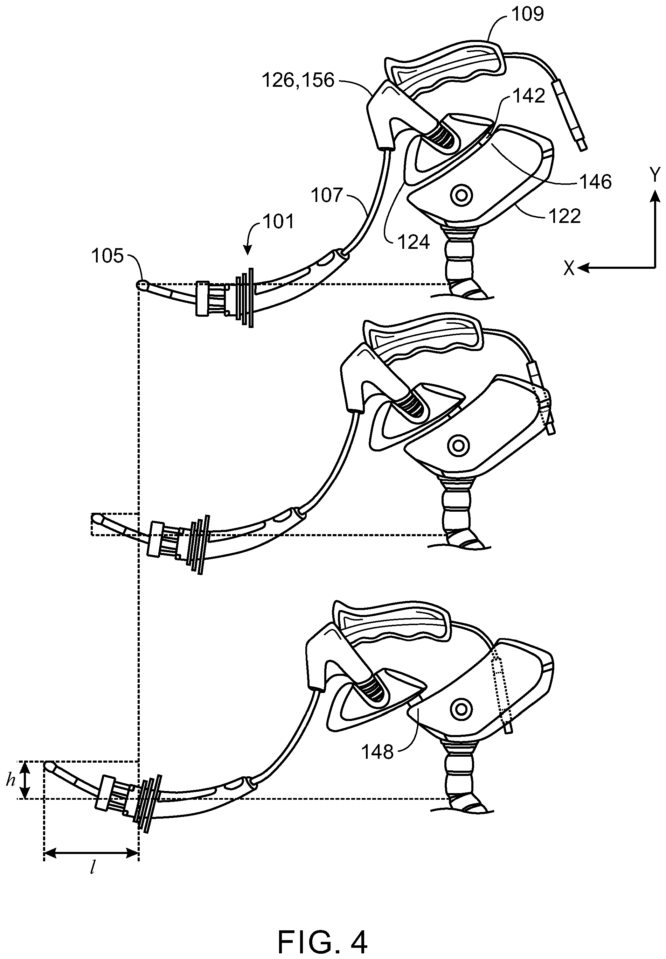

Referring still to FIGS. 2 and 3, the connector 142 is an elongate component (e.g., a rod, a peg, or an extension piece) that extends from a lower surface of the housing 138. A lower end of the connector 142 is shaped such that the lower end is retained within the elongate receptacle 136 of the base 122. The elongate receptacle 136 is formed substantially parallel to a curved profile of the housing 128 in the *xy* plane. The curved shape of the elongate receptacle 136 allows forward and backward movements of the adjustment unit 124 with respect to the horizontal x axis and upward and downward movements of the adjustment unit 124 with respect to the vertical *y* axis. The forward/backward and upward/downward components of the movement together provide the arcuate motion of the adjustment unit 124. Referring to FIG. 4, in some examples, the adjustment unit 124 can move forward (e.g., along the *x* axis) from a rearmost position 146 (e.g., defined at the connector 142) to a forward-most position 148 by a total distance *l* of up to about 6 cm to up to about 26 cm. In some examples, the adjustment unit 124 can move vertically (e.g., along the *y* axis) by a total distance *h* of up to about 5 cm to up to about 10 cm.

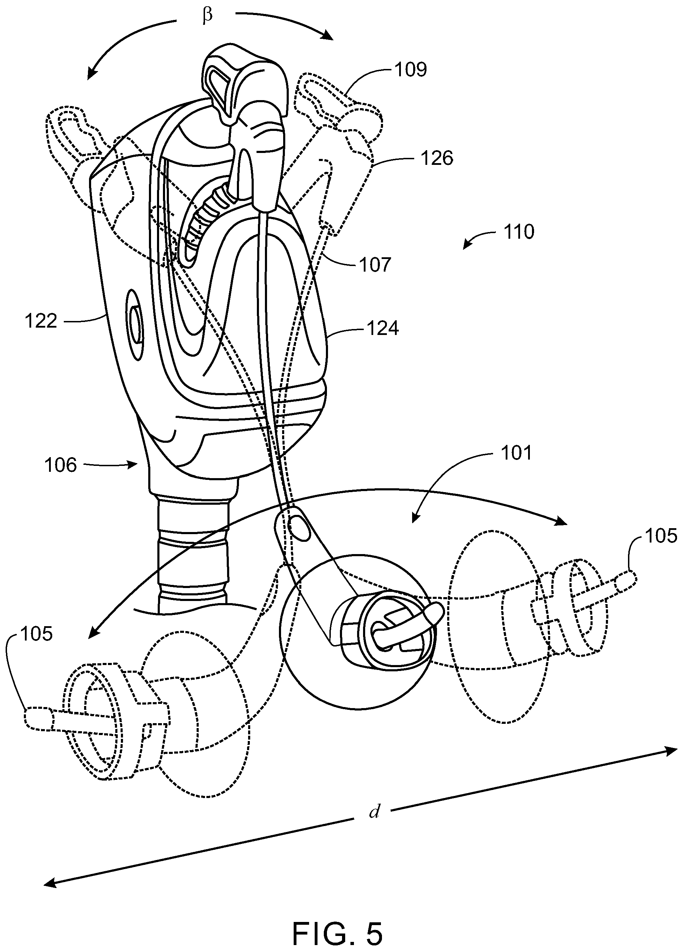

Referring to FIGS. 2 and 5, the rotational element 144 includes a dial 150 and an adapter mount. The adapter mount extends from the dial 150 and is fitted securely within a corresponding receptacle of the adapter 126. The dial 150 is rotatable about an interior pin by an angle *β* of up to about 90 degrees to up to about 270 degrees to adjust an orientation of the adapter 126 and accordingly, the orientation of the uterine manipulator 101 that is secured to the adapter 126. In some examples, such an angular span corresponds to a lateral range *d* of the tip 105 of the uterine manipulator 101 of about 6 cm to about 36 cm.

The adapter 126 includes a body 156 that defines the corresponding receptacle and an attachment feature (e.g., a slot, a receptacle, or another attachment feature) to which a shaft 107 of the uterine manipulator 101 can be securely fitted. The adapter 126 also includes a rear connection feature at which the body 156 is attachable to a handle 109 of the uterine manipulator 101. That is, the shaft 107 of the uterine manipulator 101 can be removed from the handle 109 of the uterine manipulator 101 to allow attachment of both components to the adapter 126. In some examples, the handle 109 of the uterine manipulator 101 may be discarded in lieu of a handle 109 that may instead be provided as preassembled with the body 156 of the adapter 126.

Once the uterine manipulator 101 has been installed to the adapter 126, the adapter 126 has been secured to the adjustment unit 124, and the positioning arm 104 has been locked in a desired configuration, the remote controller 108 (e.g., a joystick) can be operated at a remote user workstation to robotically manipulate the adjustment unit 124 of the adjustment device 110. For example, the adjustment unit 124 can be moved forward and downward along the elongate receptacle 136 as discussed above, where the downward movement results in an upward movement of the tip 105 of the uterine manipulator 101 due to a curved shape of the shaft 107. Additionally, the dial 150 can be rotated to change an orientation of the adapter 126 and, correspondingly, to change the orientation of the uterine manipulator 101 (e.g., to tilt the uterine manipulator 101 from side to side).

While the adjustment device 110 has been described and illustrated with respect to certain dimensions, sizes, shapes, arrangements, configurations, materials, and methods, in some examples, an adjustment device that is otherwise substantially similar in construction and function to the adjustment device 110 may include one or more different dimensions, sizes, shapes, arrangements, configurations, and materials or may be utilized according to different methods.

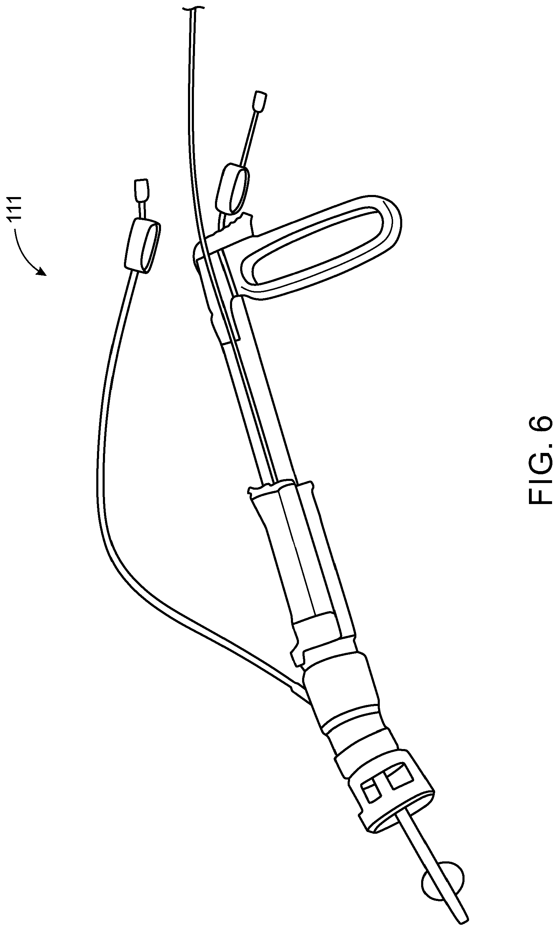

For example, while the adjustment device 110 has been described and illustrated as including the adapter 126 that is designed to mate with the uterine manipulator 101, in some examples, an adjustment device that is otherwise substantially similar in construction and function to the adjustment device 110 may alternatively include a different adapter that is designed to mate with a different uterine manipulator, such as the uterine manipulator 111 shown in FIG. 6.

While the adjustment device 110 has been described and illustrated with respect to certain numerical ranges for the parameters *l*, *h*, *β*, and *d,* in some examples, an adjustment device that is otherwise substantially similar in construction and function to the adjustment device 110 may alternatively be designed to operate according to different numerical ranges for one or more of the parameters *l*, *h*, *β*, and *d*.

While the adjustment device 110 has been described and illustrated as including the rechargeable battery 132 within the lower base 122, in some examples, an adjustment device that is otherwise substantially similar in construction and function to the adjustment device 110 may alternatively include a rechargeable battery within an upper adjustment unit instead of within a lower base.

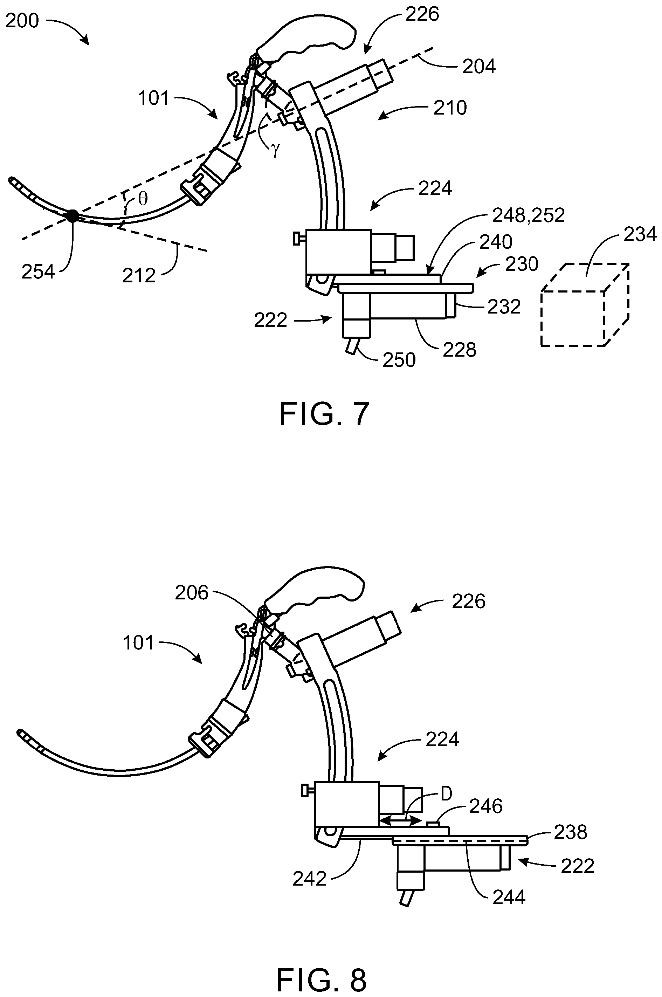

Referring to FIGS. 7 and 8, in some examples, an adjustment device 210 may be used alternatively to the adjustment device 110 for locally positioning the uterine manipulator 101 of a positioning system 200. The positioning system 200 is substantially similar in construction and function to the positioning system 100, except that the positioning system 200 includes the adjustment device 210 instead of the adjustment device 110. Accordingly, the positioning system 200 further includes the mounting/powering unit 102, the positioning arm 104, the remote controller 108, and the foot pedal 112 of the positioning system 100, all of which are shown in FIG. 1. The adjustment device 210 includes a device connector 250 by which the adjustment device 210 is fixedly attached to the positioning arm 104.

Once the positioning arm 104 of the positioning system 200 has been locked in a desired configuration to establish a global position of the adjustment device 210, the adjustment device 210 can be operated with the remote controller 108 to fine-tune a position of the uterine manipulator 101, which is supported on the adjustment device 210. The adjustment device 210 also includes a lower motor assembly 222 (e.g., a translation assembly) that is fixedly attached to the upper end 106 of the positioning arm 104, an intermediate motor assembly 224 that is fixedly attached to the lower motor assembly 222, and an upper motor assembly 226 (e.g., an adjustment unit) that is fixedly attached to the intermediate motor assembly 224.

The lower motor assembly 222 is operable to effect linear motion of the uterine manipulator 101 along the *x* axis (e.g., in both positive and negative horizontal *x*-axis directions). The motor assembly 222 includes a housing 228, a motor 232 located within the housing 228, and a translation mechanism 230 located at an upper side of the housing 228. The motor 232 drives linear motion of the translation mechanism 230. In some examples, the motor 232 is a brushless DC motor with an integrated planetary gear transmission. In some examples, one or more components of the adjustment device 210 are coupled to a separate control box 234 that supplies power to the motor 232 (e.g., via a signal cable), that communicates control signals wirelessly to the motor 232, and that effects wired or wireless communication with the remote controller 108 (shown in FIG. 1). In some examples, the adjustment device 210 alternatively includes an onboard power supply (e.g., a battery) and an onboard controller that power and control the motor 232 and that communicate with the remote controller 108.

The translation mechanism 230 includes a linear track 238 (e.g., an elongate, substantially straight track) and a linear coupling member 240 that is slidable and translatable within the linear track 238. For example, the linear coupling member 240 includes a lower rail 242 that is formed complementary to an inner profile 244 (e.g., represented by a dashed line 244) of the linear track 238 such that the rail 242 is slidable and translatable linearly along the inner profile 244. The rail 242 is connected or otherwise coupled to the motor 232 such that the linear motion of the rail 242 along the *x* direction is driven by action of the motor 232.

In some examples, the motor assembly 222 optionally includes a position stop 246 that limits an extent to which the linear coupling member 240 can translate in the +*x* direction. In some examples, the rail 242 is movable along the +*x* direction by a distance *D* of up to about 10 cm with respect to the linear track 238. The linear coupling member 240 includes an upper platform 248 from which the lower rail 242 extends and through which the position stop 246 extends. The intermediate motor assembly 224 is mounted to the platform 248 (e.g., along a substantially flat surface 252) such the that intermediate motor assembly 224 is moved linearly due to operation of the lower motor assembly 222. In this way, the lower motor assembly 222 provides the adjustment device 210 with a linear degree of freedom along the *x* axis.

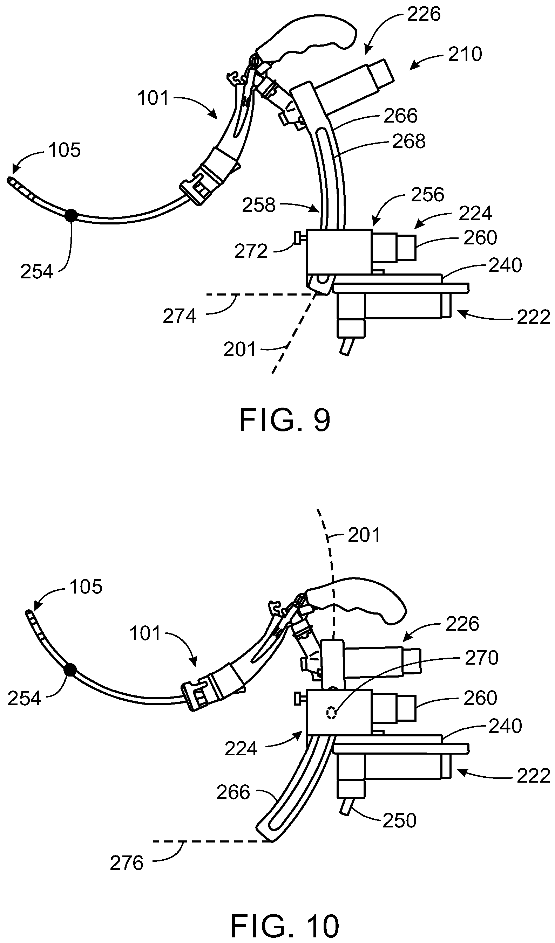

Referring to FIGS. 9 and 10, the intermediate motor assembly 224 is operable to effect arcuate motion (e.g., curvilinear motion with components along both the *x* and *y* axe) of the uterine manipulator 101 around a reference point 254. The reference point 254 is fixed with respect to the linear coupling member 240 and corresponds to a location of the cervix of the patient's uterus (e.g., a uterus of average depth of about 8 cm). The motor assembly 224 includes a housing 256 that is secured to the platform 248 of the translation mechanism 230, a motor 260 located within the housing 256, and an arcuate movement mechanism 258 that is coupled to the housing 256. The motor 260 drives arcuate motion of the mechanism 258. In some examples, the motor 260 is a brushless DC motor with an integrated planetary gear transmission. In some examples, the control box 234 (shown in FIG. 7) supplies power to the motor 260 (e.g., via a signal cable), communicates control signals wirelessly to the motor 260, and effects wired or wireless communication with the remote controller 108 (shown in FIG. 1). In alternative examples, an onboard power supply (e.g., a battery) and an onboard controller of the adjustment device 210 are provided to power and control the motor 260 and communicate with the remote controller 108.

The arcuate movement mechanism 258 includes an arcuate coupling member 266 (e.g., a support component) that defines an arcuate track 268 (e.g., a curvilinear recess, channel, or slot). The arcuate movement mechanism 258 also includes a gear 270 that is positioned within the housing 256 along the arcuate track 268 and that is coupled to and driven by the motor 260. The gear 270 is coupled to the arcuate track 268 such that operation (e.g., rotation) of the gear 270 against the arcuate track 268 causes the arcuate coupling member 266 to move upward or downward through the housing 256 along an arcuate path 201 that is constrained and defined by the arcuate track 268. The motor assembly 224 further includes a coupling member 272 that secures the arcuate coupling member 266 to the housing 256.

Movement of the arcuate coupling member 266 with respect to (e.g., through) the housing 256 along the path 201 causes the uterine manipulator 101 to undergo a corresponding arcuate motion that maintains the shaft 107 of the uterine manipulator 101 about the reference point 254 at all times. Constraining the motion of the shaft 107 in this manner ensures that (e.g., for a given x-axis position of the housing 256), the tip 105 of the uterine manipulator 101 is positioned appropriately (e.g., at a desired vertical position) within the patient's uterus. In some examples, movement of the arcuate coupling member 266 from a first vertical position 274 shown in FIG. 9 to a second vertical position 276 shown in FIG. 10 results in a vertical movement of the tip 105 through a distance of about 3 cm. A desired position (e.g., an operable position) of the tip 105 may be selected by causing the arcuate coupling member 266 to move to any desired vertical position between the first and second vertical positions 274, 276 as the arcuate coupling member 266 moves through the housing 256 along the path 201.

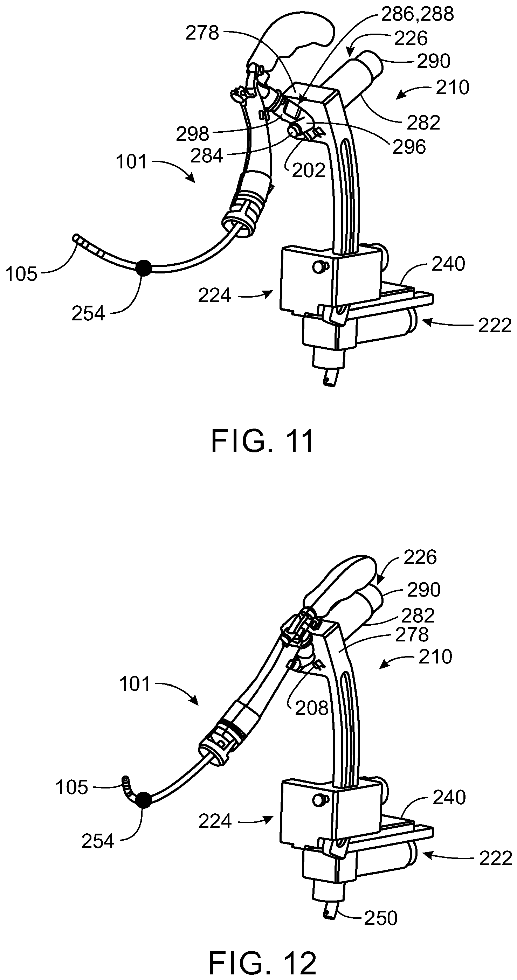

The upper motor assembly 226 is mounted to a support base 278 (shown in FIGS. 11 and 12) of the arcuate coupling member 266 (e.g., at an opening) such that the upper motor assembly 226 is moved arcuately due to operation of the intermediate motor assembly 224. In this way, the intermediate motor assembly 224 provides the adjustment device 210 with degrees of freedom along both the *x* and *y* axes by effecting arcuate motion.

Referring to FIGS. 11 and 12, the upper motor assembly 226 is operable to effect rotational motion of the uterine manipulator 101 about the reference point 254. The motor assembly 226 includes a housing 282 (e.g., a base component) that is mounted to the support base 278 of the arcuate coupling member 266. For example, a connecting rod 284 of the motor assembly 226 extends from the housing 282 through the opening in the support base 278 to a device coupling member 286 of the motor assembly 226 that is coupled to the uterine manipulator 101. The device coupling member 286 and the connecting rod 284 together provide a rotational mechanism 288. The motor assembly 226 further includes a motor 290 located within the housing 282. The motor 290 drives rotational motion of the mechanism 288. In some examples, the motor 290 is a brushless DC motor with an integrated planetary gear transmission. In some examples, the control box 234 (shown in FIG. 7) supplies power to the motor 290 (e.g., via a signal cable), communicates control signals wirelessly to the motor 290, and effects wired or wireless communication with the remote controller 108 (shown in FIG. 1). In alternative examples, an onboard power supply (e.g., a battery) and an onboard controller of the adjustment device 210 are provided to power and control the motor 290 and communicate with the remote controller 108.

The connecting rod 284 of the rotational mechanism 288 is rotatably driven by the motor 290. The device coupling member 286 is fixedly attached to and surrounds the connecting rod 284 such that rotation of the connecting rod 284 causes rotation of the device coupling member 286. The device coupling member 286 includes a substantially cylindrical portion 296 that surrounds the connecting rod 284 and a support arm 298 (e.g., a rotational element) that extends upward from the cylindrical portion 296. In some examples, the device coupling member 286 optionally includes a stopping element 202 that extends downward from the cylindrical portion 296. An adaptor 206 that is attached to the uterine manipulator 101 is mounted to the support arm 298. The support arm 298 is also oriented at an acute angle γ (refer to FIG. 7) with respect to a central axis 204 of the motor 290, which in part causes the shaft 107 of the uterine manipulator 101 to intersect the reference point 254.

Owing to the attachment of the cylindrical portion 296 to the connecting rod 284, rotation of the connecting rod 284 causes the support arm 298 to rotate correspondingly from side to side with respect to the reference point 254. In some examples, the support base 278 of the arcuate coupling member 266 optionally includes two positional stoppers 208 (only one is visible in FIGS. 11 and 12) that are located oppositely with respect to the central axis 204. The stopping element 202 abuts a positional stopper 208 when the device coupling member 286 reaches a certain degree of rotation such that the stopping elements 202 limit a rotational extent of the device coupling member 286. In some examples, the device coupling member 286 is permitted to rotate by up to about 30 degrees to each side of the housing 282 with respect to the central axis 204 of the housing 282.

Referring to FIGS. 7, 11, and 12, the central axis 204 of the motor 290 intersects the reference point 254 (e.g., corresponding to the cervix), thus ensuring that rotation of the uterine manipulator 101 occurs with respect to the cervix. An acute angle θ between the central axis 204 and a line 212 tangent to the shaft 107 of the uterine manipulator 101 and intersecting the reference point 254 is maximized to in order to maximize the rotational extent of the distal tip 105 of the uterine manipulator 101. In some examples, the angle θ is about 30 degrees to about 50 degrees. The angle θ can remain fixed because the rotational motor assembly 226 is connected directly to the uterine manipulator 101 (e.g., via the adapter 206). Mounting the motor assembly 226 in a different configuration (e.g., between the lower motor assembly 222 and the intermediate assembly 224) would undesirably cause the angle between the tangent line 212 and the central axis 204 to change as the arcuate motion (discussed with respect to FIGS. 9 and 10) of the uterine manipulator 101 changes, which is unacceptable during a surgical procedure.

The upper motor assembly 224 provides the adjustment device 210 with a rotational degree of freedom about the reference point 254. In some examples, the control box 234 (shown in FIG. 7) supplies power to the motor 290, communicates control signals wirelessly to the motor 290, and effects wired or wireless communication with the remote controller 108 (shown in FIG. 1). In alternative examples, an onboard power supply (e.g., a battery) and an onboard controller of the adjustment device 210 are provided to power and control the motor 290 and communicate with the remote controller 108.

In some examples, the adjustment device 210 may be powered on and off according to operation of the control box 234, and the adjustment device 210 may still optionally include a power selector (e.g., a button or a switch) located on the housing 228 or another component of the adjustment device 210. In alternative examples for which the adjustment device 210 includes an onboard power supply (e.g., a battery) and an onboard controller, the adjustment device 210 includes a power selector located on the housing 228 or another component of the adjustment device 210. In some examples, the adjustment device 210 weighs about 2 kg to about 4 kg. Example materials from which the housings 228, 256, 282 and components of the mechanisms 230, 258, 288 may be made include steel and aluminum.

While the adjustment device 210 has been described and illustrated with respect to certain dimensions, sizes, shapes, arrangements, configurations, materials, and methods, in some examples, an adjustment device that is otherwise substantially similar in construction and function to the adjustment device 210 may include one or more different dimensions, sizes, shapes, arrangements, configurations, and materials or may be utilized according to different methods.

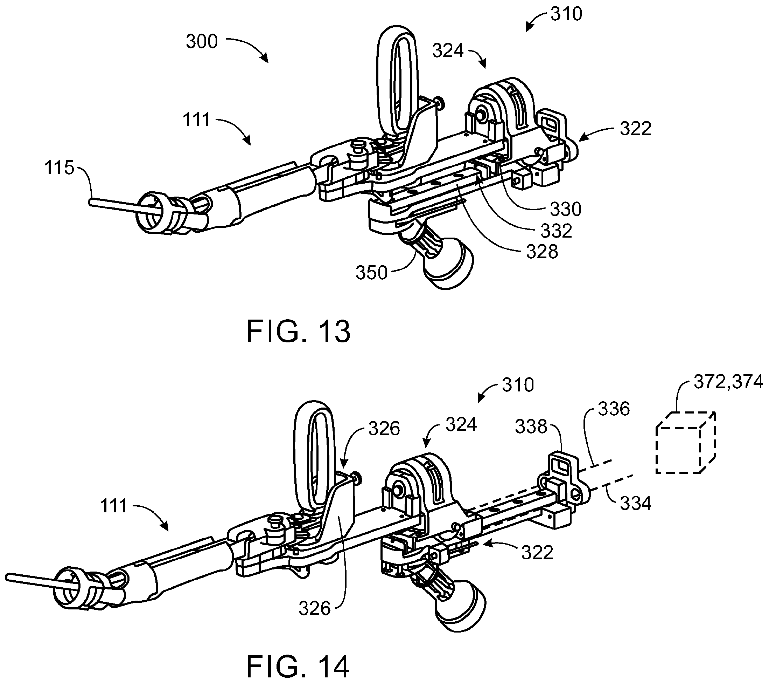

Referring to FIGS. 13 and 14, in some embodiments, an adjustment device 310 may be used for locally positioning the uterine manipulator 111 of a positioning system 300. The positioning system 300 is substantially similar in construction and function to the positioning system 100, except that the positioning system 300 includes the adjustment device 310 instead of the adjustment device 110 and the uterine manipulator 111 instead of the uterine manipulator 101. Accordingly, the positioning system 300 further includes the mounting/powering unit 102, the positioning arm 104, the remote controller 108, and the foot pedal 112 of the positioning system 100, all of which are shown in FIG. 1.

Once the positioning arm 104 of the positioning system 300 has been locked in a desired configuration to establish a global position of the adjustment device 310, the adjustment device 310 can be operated with the remote controller 108 to fine-tune a position of the uterine manipulator 111, which is supported on the adjustment device 310. Accordingly, the adjustment device 310 includes a device connector 350 by which the adjustment device 310 is fixedly attached to the positioning arm 104. The adjustment device 310 also includes a translational assembly 322 that is fixedly attached to the upper end 106 of the positioning arm 104, a rotational assembly 324 that is fixedly attached to the translational assembly 322, and a rotatable receptacle 326 (e.g., a rotatable adjustment unit) that is fixedly attached to the rotational assembly 324.

The translational assembly 322 is operable to effect linear motion of the uterine manipulator 111 along the x axis (e.g., in both positive and negative horizontal x-axis directions). The translational assembly 322 includes a rail 328 that is fixed to the device connector 350, a rear wall 338 that is fixed to an end of the rail 328, and a horizontal support structure 330 (e.g., a carriage) that is slidable linearly along the rail 328. Accordingly, the rail 328 has a substantially rectangular shape, and the horizontal support structure 330 defines a slot 332 with a complementary shape to allow linear movement of the horizontal support structure 330 along the rail 328.

The translational assembly 322 further includes two flexible mechanical actuation cables (e.g., Bowden cables) by which the horizontal support structure 330 can be moved (e.g., pushed forward) in the +*x* direction by one cable 334 (represented by the dashed line 334) and moved (e.g., pulled backward) in the -*x* direction by the other cable 336 (represented by the dashed line 336). Each cable 334, 336 is connected to the horizontal support structure 330 at one end and passes through the rear wall 338 to connect to a remote motor 372 at an opposite end. The remote motor 372 is controlled by associated electronics 374 upon operation of the remote controller 108 to activate the cables 334, 336 for moving the horizontal support structure 330 linearly. In some embodiments, the remote motor 372 is a linear actuator. In some embodiments, the horizontal support structure 330 is movable along the +x direction by a distance of up to about 10 cm with respect to the rail 328. In this way, the translational assembly 322 provides the adjustment device 310 with a linear degree of freedom along the x axis.

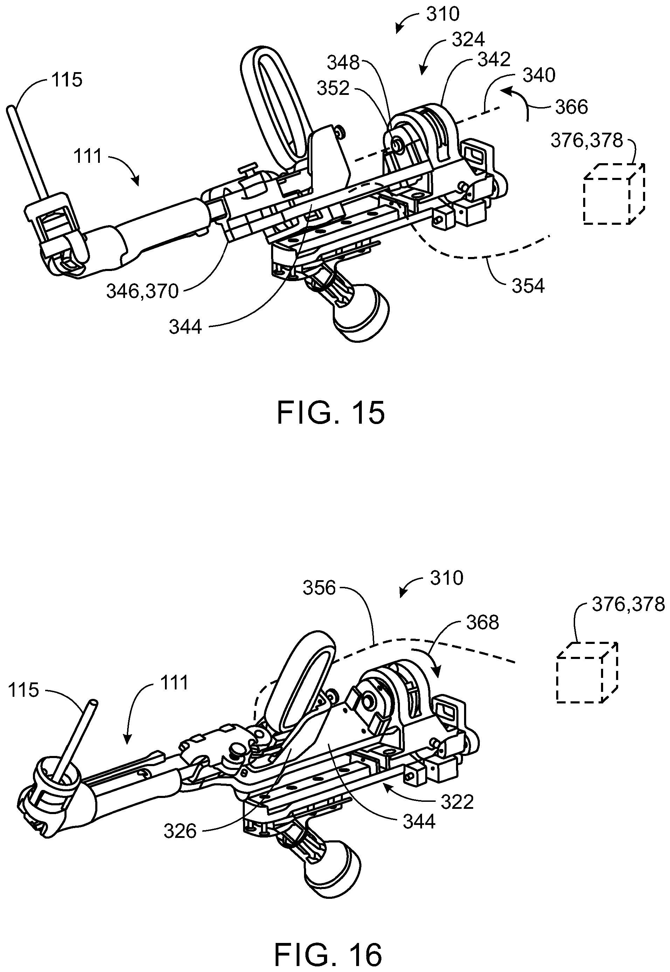

Referring to FIGS. 15 and 16, the rotational assembly 324 that is operable to effect rotational motion of the uterine manipulator 111 about a rotational axis 340 that extends parallel to the x axis and through the patient's cervix. The rotational assembly 324 includes a vertical support structure 342 and a platform 344 (e.g., a rotatable support component) that is coupled to the vertical support structure 342. The platform 344 includes a base 346 that is secured to the uterine manipulator 111 at a distal portion 370 and a coupling wall 348. The rotational assembly 324 includes a pin 352 that couples the coupling wall 348 of the platform 344 to the vertical support structure 342 of the translational assembly 322 to allow the platform 344 to rotate (e.g., pivot) with respect to the translational assembly 322.

The rotational assembly 324 further includes two flexible mechanical actuation cables 354, 356 (e.g., Bowden cables) by which the platform 344 can be rotated from side to side with respect to the rotational axis 340. The flexible cable 354 (represented by the dashed line 354 in FIG. 14) is connected to one side of the platform 344 and can be pulled to move the platform 344 in a first rotational direction 358. The flexible cable 356 (represented by the dashed line 356 in FIG. 13) is connected to the opposite side of the platform 344 and can be pulled to move the platform 344 in a second, opposite rotational direction 360.

Each cable 354, 356 is connected to the platform 346 at one end and is connected to a remote motor 376 at an opposite end. The remote motor 376 is controlled by associated electronics 378 upon operation of the remote controller 108 to activate the cables 354, 356 for rotating the platform 346 about the pin 352. In some embodiments, the remote motor 376 is a linear actuator. In some embodiments, the platform 344 is rotatable to each side of the rotational axis 340 by an angle of up to about 90 degrees. The rotatable receptacle 326 is rotatably coupled to the platform 344. In this way, the rotational assembly 324 provides the adjustment device 310 with a rotational degree of freedom with respect to the rotational axis 340, which extends parallel to the x axis.

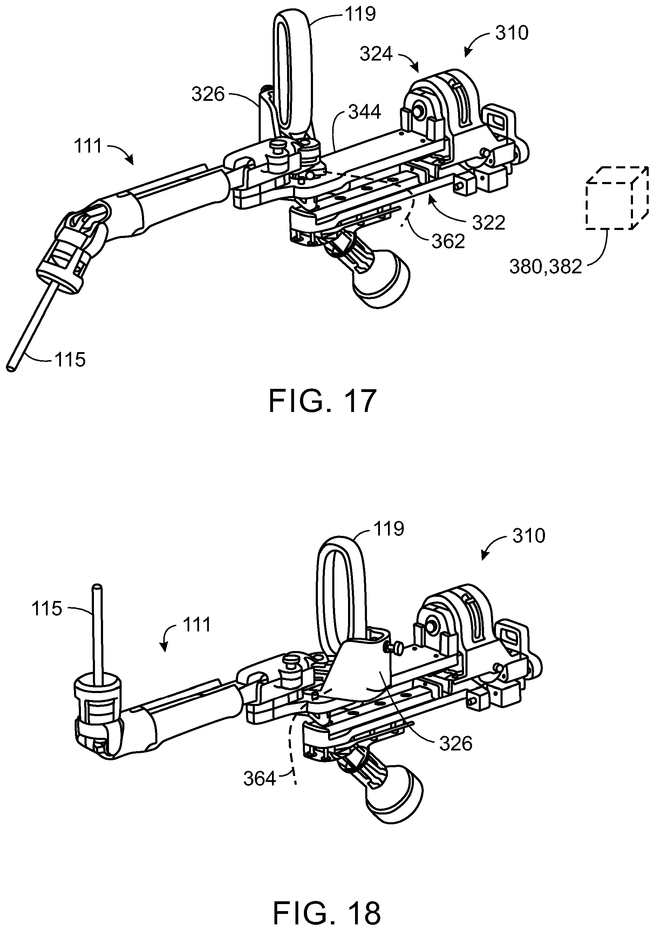

Referring to FIGS. 17 and 18, the rotatable receptacle 326 is operable to effect rotational motion of a distal tip 115 of the uterine manipulator 111 about a transverse axis 123 that extends perpendicular (e.g., along a z axis) to a central axis 117 of the uterine manipulator 111. The distal tip 115 can be articulated (e.g., pivoted) upward by about 90 degrees (shown in FIG. 18) about the transverse axis 123 and downward by about 50 degrees (shown in FIG. 17) about the transverse axis 123 by rotating a handle 119 (e.g., an articulation control element) of the uterine manipulator 111 side to side with respect to the central axis 117 at a pin mechanism 121.

The rotatable receptacle 326 provides a seat (e.g., a holding or support component) in which the handle 119 is securely mounted for rotating the handle 119. The receptacle 326 is equipped with two flexible mechanical actuation cables 362, 364 (e.g., Bowden cables) by which the receptacle 326, carrying the handle 119, can be rotated from side to side with respect to the central axis 117. The flexible cable 362 (represented by the dashed line 362 in FIG. 17) is connected to one side of the receptacle 326 and can be pulled to rotate the receptacle in a first rotational direction 366. The flexible cable 364 (represented by the dashed line 364 in FIG. 18) is connected to the opposite side of the receptacle 326 and can be pulled to rotate the receptacle 326 in a second, opposite rotational direction 368.

Each flexible cable 362, 364 is connected to the receptacle 326 at one end and is connected to a remote motor 380 at an opposite end. The remote motor 380 is controlled by associated electronics 382 upon actuation of the remote controller 108 to activate the cables 362, 364 for rotating the receptacle 326 about the pin mechanism 121. In some embodiments, the remote motor 380 is a linear actuator. In this way, the rotatable receptacle 326 provides the adjustment device 310 with a rotational degree of freedom about the transverse axis 123, which extends perpendicular to the central axis 117 and to the x axis.

In contrast to the uterine manipulator 101, the uterine manipulator 111 includes different distal tips 115 that can be selected for use with uteruses of differing depths. The result is that the cervix remains in the same position relative to the handle 119 such that the adjustment device 310 can adjust the distal tip 115 via the rotational assembly 324 and the rotatable receptacle 326 at the cervix irrespective of the depth of the uterus.

Location of the motors 372, 376, 380 of the adjustment device 310 at remote positions advantageously reduces a weight of the adjustment device 310. In some embodiments, the adjustment device 310 weighs about 0.5 kg to about 3 kg. Example materials from which the translational assembly 322, the rotational assembly 324, and the rotatable receptacle 326 may be made include aluminum and steel.

While the adjustment device 310 has been described and illustrated with respect to certain dimensions, sizes, shapes, arrangements, configurations, materials, and methods, in some embodiments, an adjustment device that is otherwise substantially similar in construction and function to the adjustment device 310 may include one or more different dimensions, sizes, shapes, arrangements, configurations, and materials or may be utilized according to different methods.

For example, while the adjustment device 310 has been described and illustrated as including the rotatable receptacle 326 that is designed to seat the handle 119 of the uterine manipulator 111, in some embodiments, an adjustment device that is otherwise substantially similar in construction and function to the adjustment device 310 may alternatively include a rotatable receptacle 326 that is designed to seat a different rotatable component (e.g., a fitting) of the uterine manipulator 111 that replaces the handle 119.

While the adjustment device 310 has been described and illustrated as including remote motors 372, 376, 380 that provide actuation via the mechanical actuation cables 334, 336, 354, 356, 362, 364, in some embodiments, an adjustment device that is otherwise substantially similar in construction and function to the adjustment device 310 may alternatively include remote motors that provide actuation via hydraulic mechanisms.

While the adjustment device 310 has been described and illustrated as including remote motors 372, 376, 380 that provide actuation via the mechanical actuation cables 334, 336, 354, 356, 362, 364, in some embodiments, an adjustment device that is otherwise substantially similar in construction and function to the adjustment device 310 may alternatively include one or more local motors that are connected directly to local components of the assemblies 322, 324 and to the receptacle 326.

The adjustment device 310 has been described and illustrated as including three sets of two mechanical actuation cables 334, 336; 354, 356; 362, 364, where each cable of each set effects an oppositely directed movement of the mechanism 322, 324, 326, respectively. However, in some embodiments, an adjustment device that is otherwise substantially similar in construction and function to the adjustment device 310 may alternatively include a single flexible mechanical actuation cable for each movement mechanism 322, 324, 326 and that can selectively effect either of two oppositely directed movements. In some embodiments, either cable of a set of two mechanical actuation cables 334, 336; 354, 356; or 362, 364 may be configured to effect either of two alternative oppositely directed movements.

Remotely controlling robotic movements of the adjustment devices 110, 210, 310 can allow the surgeon to position the patient's uterus (e.g., seated on a uterine manipulator 101, 111, which is in turn secured to an adjustment device 110, 210, 310) as desired for optimal cutting and removal access to the uterus without leaving his or her workstation at which the remote controller 108 is operated. Thus, the surgeon can continue visualizing the patient's pelvic region on a display screen at the robotic surgery workstation while the adjustment device 110, 210, 310 is manipulated remotely without disruption. Such remote control of the adjustment devices 110, 210, 310 therefore advantageously results in a streamlined procedure with an improved effectiveness, an improved precision, and a reduced procedural time period.

In some examples and embodiments, any of the adjustment devices 110, 210, 310 may be provided as a modular device that can be retrofitted to or otherwise assembled with a positioning arm 104 of a positioning system 100, 200, 300 at a point of care. In other examples and embodiments, the adjustment device 110, 210, 310 may be preassembled with a positioning arm 104 of a positioning system 100, 200, 300.

While the adjustment devices 110, 210, 310 have been described and illustrated as permitting certain degrees of freedom, in some examples and embodiments, an adjustment device that is otherwise substantially similar in construction and function to any of the adjustment devices 110, 210, 310 may be designed to allow additional movements and/or degrees of freedom.

Accordingly, other embodiments are also possible as long as they are within the scope of the following claims.

## Claims

1. An adjustment device (310) for positioning a uterine manipulator (111), the adjustment device (310) comprising:
a linear translation assembly (322) comprising a rail (328) and a carriage (330) that is movable in a horizontal axis direction;
**characterized by** further comprising:
a rotatable support component (344) that is configured to be secured to the uterine manipulator (111), rotatable with respect to the linear translation assembly (322), and rotatable from side to side with respect to a rotational axis (340) defined by the adjustment device (310), wherein the rotational axis (340) extends parallel to the horizontal axis direction, and wherein the rotational axis (340) intersects a patient's cervix; and
a rotatable receptacle (326) that is configured to be secured to an articulation control element (119) of the uterine manipulator (111) and rotatably coupled to the rotatable support component (344) such that the adjustment device (310) is configured to rotate the uterine manipulator (111) with respect to the rotational axis (340) and to articulate a portion of the uterine manipulator (111) with respect to the rotatable support component (344).

2. The adjustment device (310) of claim 1, wherein the rotatable receptacle (326) provides a seat in which the articulation control element (119) is securely mounted for rotating the articulation control element (119).

3. The adjustment device (310) of claim 1, wherein the carriage (330) is slidable linearly along the rail (328).

4. The adjustment device (310) of claim 1, wherein the linear translation assembly (322) is configured to be fixedly attached to a positioning arm (104) of a uterine positioning system (300).

5. The adjustment device (310) of claim 1, further comprising one or more mechanical actuation cables (354, 356) that are configured to selectively cause the rotatable support component (344) to rotate in opposite rotational directions.

6. The adjustment device (310) of claim 5, further comprising a remote motor (372) that is coupled to and controls the one or more mechanical actuation cables (354, 356).

7. The adjustment device (310) of claim 1, further comprising one or more mechanical actuation cables (362, 364) that are configured to selectively cause the rotatable receptacle (326) to rotate in opposite rotational directions.

8. The adjustment device (310) of claim 7, further comprising a remote motor (376) that is coupled to and controls the one or more mechanical actuation cables (362, 364).

9. The adjustment device (310) of claim 1, wherein the articulation control element (119) comprises a handle (119), and wherein the portion comprises a distal tip (115).

10. A positioning system (300) comprising:
a positioning arm (104); and
the adjustment device (310) of claim 2, wherein the adjustment device (310) is supported on the positioning arm (104) for positioning the uterine manipulator (111).

11. The positioning system (300) of claim 10, wherein the positioning arm (104) defines a global position of the adjustment device (310), and wherein the adjustment device (310) defines a local position of the uterine manipulator (111).

## Patentansprüche

1. Einstellvorrichtung (310) zur Positionierung eines Gebärmuttermanipulators (111), wobei die Einstellvorrichtung (310) umfasst:
eine Anordnung zur linearen Translation (322), die eine Schiene (328) und einen Wagen (330) umfasst, der in eine horizontale Achsrichtung beweglich ist;
**gekennzeichnet durch** ferner umfassend:
eine drehbare Stützkomponente (344), die dazu ausgelegt ist, an dem Gebärmuttermanipulator (111) befestigt zu werden, die mit Bezug auf die Anordnung zur linearen Translation (322) drehbar ist und mit Bezug auf eine Drehachse (340), die durch die Einstellvorrichtung (310) definiert wird, von Seite zu Seite drehbar ist, wobei sich die Drehachse (340) parallel zur horizontalen Achsrichtung erstreckt, und wobei die Drehachse (340) den Gebärmutterhals einer Patientin schneidet; und
eine drehbare Aufnahme (326), die dazu ausgelegt ist, an einem Auslenksteuerelement (119) des Gebärmuttermanipulators (111) befestigt und drehbar mit der drehbaren Stützkomponente (344) gekoppelt zu werden, so dass die Einstellvorrichtung (310) dazu ausgelegt ist, den Gebärmuttermanipulator (111) mit Bezug auf die Drehachse (340) zu drehen und einen Abschnitt des Gebärmuttermanipulators (111) mit Bezug auf die drehbare Stützkomponente (344) auszulenken.

2. Einstellvorrichtung (310) nach Anspruch 1, wobei die drehbare Aufnahme (326) einen Sitz bereitstellt, in dem das Auslenksteuerelement (119) zum Drehen des Auslenksteuerelements (119) fest montiert ist.

3. Einstellvorrichtung (310) nach Anspruch 1, wobei der Wagen (330) entlang der Schiene (328) linear verschiebbar ist.

4. Einstellvorrichtung (310) nach Anspruch 1, wobei die Anordnung zur linearen Translation (322) dazu ausgelegt ist, fest an einem Positionierarm (104) eines Gebärmutterpositioniersystems (300) angebracht zu werden.

5. Einstellvorrichtung (310) nach Anspruch 1, ferner umfassend ein oder mehrere mechanische Betätigungskabel (354, 356), die dazu ausgelegt sind, gezielt zu bewirken, dass sich die drehbare Stützkomponente (344) in entgegengesetzte Drehrichtungen dreht.

6. Einstellvorrichtung (310) nach Anspruch 5, ferner umfassend einen entfernten Motor (372), der mit dem einen oder den mehreren mechanischen Betätigungskabeln (354, 356) gekoppelt ist und diese(s) steuert.

7. Einstellvorrichtung (310) nach Anspruch 1, ferner umfassend ein oder mehrere mechanische Betätigungskabel (362, 364), die dazu ausgelegt sind, gezielt zu bewirken, dass sich die drehbare Aufnahme (326) in entgegengesetzte Drehrichtungen dreht.

8. Einstellvorrichtung (310) nach Anspruch 7, ferner umfassend einen entfernten Motor (376), der mit dem einen oder den mehreren mechanischen Betätigungskabeln (362, 364) gekoppelt ist und diese(s) steuert.

9. Einstellvorrichtung (310) nach Anspruch 1, wobei das Auslenksteuerelement (119) einen Griff (119) umfasst und wobei der Abschnitt eine distale Spitze (115) umfasst.

10. Positioniersystem (300), umfassend:
einen Positionierarm (104); und
die Einstellvorrichtung (310) nach Anspruch 2, wobei die Einstellvorrichtung (310) zum Positionieren des Gebärmuttermanipulators (111) auf dem Positionierarm (104) gestützt ist.

11. Positioniersystem (300) nach Anspruch 10, wobei der Positionierarm (104) eine globale Position der Einstellvorrichtung (310) definiert und wobei die Einstellvorrichtung (310) eine lokale Position des Gebärmuttermanipulators (111) definiert.

## Revendications

1. Dispositif de réglage (310) pour positionner un manipulateur utérin (111), le dispositif de réglage (310) comprenant :
un ensemble de translation linéaire (322) comprenant un rail (328) et un chariot (330) qui est mobile dans une direction d'axe horizontal ;
**caractérisé en ce qu'**il comprend en outre :
un composant de support rotatif (344) qui est configuré pour être fixé au manipulateur utérin (111), rotatif par rapport à l'ensemble de translation linéaire (322), et rotatif d'un côté à l'autre par rapport à un axe de rotation (340) défini par le dispositif de réglage (310), l'axe de rotation (340) s'étendant parallèlement à la direction de l'axe horizontal, et l'axe de rotation (340) coupant le col du patient ; et
un réceptacle rotatif (326) qui est configuré pour être fixé à un élément de commande d'articulation (119) du manipulateur utérin (111) et accouplé rotatif au composant de support rotatif (344) de telle sorte que le dispositif de réglage (310) soit configuré pour faire tourner le manipulateur utérin (111) par rapport à l'axe de rotation (340) et pour articuler une partie du manipulateur utérin (111) par rapport au composant de support rotatif (344).

2. Dispositif de réglage (310) selon la revendication 1, dans lequel le réceptacle rotatif (326) fournit un siège dans lequel l'élément de commande d'articulation (119) est solidement monté pour faire tourner l'élément de commande d'articulation (119).

3. Dispositif de réglage (310) selon la revendication 1, dans lequel le chariot (330) peut coulisser linéairement le long du rail (328).

4. Dispositif de réglage (310) selon la revendication 1, dans lequel l'ensemble de translation linéaire (322) est configuré pour être fixé à demeure à un bras de positionnement (104) d'un système de positionnement utérin (300).

5. Dispositif de réglage (310) selon la revendication 1, comprenant en outre un ou plusieurs câbles d'actionnement mécanique (354, 356) qui sont configurés pour provoquer sélectivement la rotation du composant de support rotatif (344) dans des directions de rotation opposées.

6. Dispositif de réglage (310) selon la revendication 5, comprenant en outre un moteur à distance (372) qui est accouplé au ou aux câbles d'actionnement mécaniques (354, 356) et commande ceux-ci.

7. Dispositif de réglage (310) selon la revendication 1, comprenant en outre un ou plusieurs câbles d'actionnement mécanique (362, 364) qui sont configurés pour provoquer sélectivement la rotation du réceptacle rotatif (326) dans des directions de rotation opposées.

8. Dispositif de réglage (310) selon la revendication 7, comprenant en outre un moteur à distance (376) qui est accouplé au ou aux câbles d'actionnement mécaniques (362, 364) et commande ceux-ci.

9. Dispositif de réglage (310) selon la revendication 1, dans lequel l'élément de commande d'articulation (119) comprend une poignée (119), et dans lequel la partie comprend une pointe distale (115).

10. Système de positionnement (300) comprenant :
un bras de positionnement (104) ; et
le dispositif de réglage (310) selon la revendication 2, dans lequel le dispositif de réglage (310) est supporté sur le bras de positionnement (104) pour positionner le manipulateur utérin (111).

11. Système de positionnement (300) selon la revendication 10, dans lequel le bras de positionnement (104) définit une position globale du dispositif de réglage (310), et dans lequel le dispositif de réglage (310) définit une position locale du manipulateur utérin (111).
